# EUROPÄISCHE PATENTANMELDUNG

(11) **EP 2 281 587 A1**
(43) Veröffentlichungstag der Anmeldung: **09.02.2011**
(21) Anmeldenummer: 09167043.0
(22) Anmeldetag: 03.08.2009
(51) Int. Cl.: A61L 15/42, A61L 15/60

(54) **Resorbierbare Polyurethanschaum-Wundabdeckung**

(71) Anmelder: nolax AG, 6203 Sempach Station (CH)
(72) Erfinder: Blume, Jessica. Dr, 8050 Zürich (CH); Buser, Stefan. Dr, 6210 Sursee (CH); Dobmann, Andreas, 6208 Obenkirch (CH); Zimmermann, Erika, 6374 Buochs (CH)
(74) Vertreter: Hepp, Dieter

(57) **Zusammenfassung**

Die Erfindung bezieht sich auf eine biokompatible, vom Körper resorbierbare, offenporige Polyurethanschaum-Wundabdeckung mit einstellbarer Resorptionsgeschwindigkeit und verbesserten Anwendungseigenschaften bei Haut- und Bindegewebsverletzungen sowie ein Verfahren zur Herstellung einer solchen Polyurethanschaum-Wundabdeckung.

## Beschreibung

Die vorliegende Erfindung betrifft eine biokompatible, vom Körper resorbierbare, offenporige Polyurethanschaum-Wundabdeckung mit einstellbarer Resorptionsgeschwindigkeit und verbesserten Anwendungseigenschaften bei Haut- und Bindegewebsverletzungen sowie ein Verfahren zur Herstellung einer solchen Polyurethanschaum-Wundabdeckung. Die individuell konfektionierbare, offenporige Polyurethanschaum-Wundabdeckung wird auf eine Wunde oder in eine Wundhöhle gegeben und bildet dort eine Adhäsionsgrundlage für in das Wundbett neu einwachsende Zellen. Die Geschwindigkeit, mit der die Polyurethanschaum-Wundabdeckung vom Körper resorbiert wird, lässt sich den Wundeigenschaften anpassen.

Die Verwendung von Polyurethanschäumen in der Medizin und insbesondere zur aufliegenden Wundabdeckung ist bekannt. Bei der Wundversorgung können Schäume die Wunde vor Austrocknung schützen, Wundexsudat aufsaugen, als Matrix für Wirkstoffe aller Art dienen und auch als Basis für die Besiedelung mit Hautzellen dienen.

Im Sinne der Anmeldung ist unter Wundabdeckung ein Material zu verstehen, welches sowohl zur aufliegenden Abdeckung einer Wunde geeignet ist (topische Applikation) als auch in eine Wundhöhle gegeben werden kann.

Wundabdeckungen aus Polyurethanschaum sind in verschiedenen Varianten erhältlich. Einige sind selbstklebend, während die meisten eine externe Fixierung, z.B. mittels Klebeband oder einem Gazeverband benötigen. Schäume werden unter anderem als Platten oder Rollen verschiedner Dicken angeboten. Durch Zurechtschneiden können diese der jeweiligen Wundgrösse angepasst werden (R.A. Bryant, D.P. Nix, Acute and chronic wounds: current management concepts, Elsevier Health Sciences, 2007, Kapitel 19, Seite 411f).

Beispielsweise beschreiben die US 3,978,266, US 3,975,567 sowie die EP 0 059 048 Polyurethanschäume zur aufliegenden Wundbehandlung.

Die WO 88/01878 beschreibt selbstklebende Schäume aus Polyurethan, welche einpolymerisierte Methacrylate enthalten können. Der Schaum kann zur Verwendung als Wundpflaster auf eine andere Polymerfolie aufgetragen werden. Weiter wird die Möglichkeit besprochen, dass Wirkstoffe, wie beispielsweise Antibiotika oder Wachstumsfaktoren, in solche Pflaster eingebracht werden.

Die EP 1 981 550 beschreibt ein Verfahren zum Auftragen einer Keimbarriere aus einem Folienmaterial auf ein Schaumstoffmaterial und eine daraus hergestellte Wundabdeckung. Dabei wird ein thermoplastisches Material direkt auf das Schaumstoffmaterial gesprüht bez. extrudiert.

Ein wesentlicher Nachteil dieser Polyurethanschäume ist, dass sie nicht resorbierbar sind und daher immer vollständig vom Körper entfernt werden müssen und somit beim Verbandswechsel neu einwachsende Zellen aus dem Wundbett abgerissen werden. Ein resorbierbarer Polyurethanschaum wie in der vorliegenden Erfindung kann als Adhäsionsgrundlage für in das Wundbett neu einwachsende Zellen im Sinne einer extrazellulären Matrix dienen, wodurch die Wundheilung beschleunigt wird.

Ein Polyurethanschaum wird vom Körper resorbiert, wenn der Schaum durch Hydrolyse, Oxidation oder enzymatisch in kleinere Bestandteile gespalten werden kann. Zur Hydrolyse kommt es, da der Schaum einem wässrigem Medium, insbesondere Wundexsudat ausgesetzt ist. Oxidationsreaktionen werden durch freie Sauerstoff-Radikale ausgelöst, welche unter anderem durch Makrophagen bei einer Entzündung freigesetzt werden. Makrophagen setzten ausserdem das Enzym Cholesterolesterase frei, welches Esterbindungen in einem Polyurethanschaum spaltet. Als Hauptabbaumechanismus von resorbierbaren Biopolymeren wird die Hydrolyse angesehen (S.A. Guelcher, Biodegradable Polyurethanes: Synthesis and Applications in Regenerative Medicine, Tissue Engineering: Part B, 2008, Volume 14, Number 1; E.M. Christenson, S. Patel, J.M. Anderson, A. Hiltner, Enzymatic degradation of poly(ether urethane) and poly(carbonate urethane) by cholesterol esterase, Biomaterials, 2006, Volume 23, 3920 - 3926).

Die US 2007/0299151 beschreibt einen biokompatiblen und resorbierbaren Polyurethanschaum. Dabei werden biokompatible Polyole, vor allem Polycaprolactone, mit mindestens einem Isocyanat, Wasser, einem oder mehreren Stabilisatoren sowie einem Porenöffner zu einem Schaum reagiert. Dieser Polyurethanschaum soll in flüssiger Form in eine Knochenfraktur gegeben werden und dort zu einem offenporigen, langsam resorbierbaren Schaum ausreagieren. An einen Polyurethanschaum, der als artifizielle extrazelluläre Matrix für Knochengewebe eingesetzt wird, werden andere Anforderungen gestellt, als an einen Schaum der bei Haut- oder Bindegewebsverletzungen eingesetzt werden soll. Polyurethanschäume, die als Füllstoffe für Knochenfrakturen eingesetzt werden, sollten einen Druckwiderstand von 0.05 - 100 MPa aufweisen und somit die Härte und Festigkeit des Knochengewebes simulieren können. Auch die Resorptionsgeschwindigkeit muss der langsameren Zellwachstumsgeschwindigkeit im Knochen angepasst sein, da eine akute Haut- oder Bindegewebsverletzung 2 bis 3 Wochen, eine Fraktur des Femurs ca. 8 bis 12 Wochen bis zu bis zu vollständigen Ausheilung benötigt.

Für die Verwendung eines offenporigen Polyurethanschaums als artifizielle extrazelluläre Matrix bei Haut- oder Bindegewebsverletzungen gelten andere Anforderungen. Der Schaum muss weich sein (möglichst <0.005 MPa im nassen Zustand) und sich dem Wundbett anpassen können. Ausserdem sollte die Resorptionsgeschwindigkeit der Zellwachstumsgeschwindigkeit der Hautzellen angepasst sein, also im Bereich von zwei Tagen bis vier Wochen liegen können.

Nach der DIN EN ISO 7726 werden Schäume nach ihrer Härte und der Eigenschaft ihrer Zellen eingeteilt. Eine Zelle ist demnach der bei der Herstellung von Schaumstoff einzeln gebildete kleine Hohlraum, der von Zellwänden und/ oder Zellstegen teilweise oder vollständig umschlossen ist (DIN EN ISO 7726). Eine offene Zelle ist folglich eine Zelle, die über die Gasphase mit anderen Zellen in Verbindung steht. Der Volumenanteil offener und geschlossener Poren ist nach DIN EN ISO 4590 bestimmbar.

Bei Wunden unterscheidet man akute und chronische Wunden. Akute Wunden sind Verletzungen und postoperative Wunden, die in einer normalen Geschwindigkeit heilen. Stagnieren Wunden in der Wundheilung und zeigen nach sechs bis acht Wochen Behandlung noch keine Heilungsverbesserung, so spricht man von chronischen Wunden. Chronische Wunden sind meist Sekundärerkrankungen bei Patienten mit Diabetes mellitus oder Herzkreislauferkrankungen. Die häufigsten chronischen Wunden sind Ulcus cruris venosum, aterielles Ulcus, diabetisches Fusssyndrom und Dekubitus. Dabei variieren chronische Wunden stark in ihrer Grösse und Tiefe, können infiziert sein und bilden unterschiedliche Mengen an Exsudat. Besonders beim Dekubitus bilden sich oft tiefe, vom Pflegepersonal nicht einsehbare Wundhöhlen, da sich die einzelnen Gewebeschichten voneinander abgelöst haben. Durch den Verbandswechsel kann das Pflegepersonal den Zustand der Wunde einschätzen und über die weitere Behandlung entscheiden. Bei stark exsudierenden Wunden muss der Verband mehrmals täglich, bei schwach exsudierenden nur einmal pro Woche gewechselt werden.

Die WO 2006/032501 beschreibt einen offenporigen Polyurethanschaum ohne Hautbildung, der als Trägermaterial für Zell- und Gewebekulturen oder Arzneimittel verwendet werden kann. Die Offenporigkeit des Schaums in der WO 2006/032501 wird durch die Verwendung von einer Polysaccharidkomponente in einer Menge von 0.01 - 4.2 Gewichtsprozent erreicht.

Bei Implantaten und resorbierbaren Materialien besteht die Gefahr der Biofilm Bildung durch Mikroorganismen. Die anfängliche Adhäsion der meisten pathogenen Mikroorganismen an Körperzellen erfolgt durch eine Bindung an die Zuckerseitenketten der Glykoproteine auf der Zelloberfläche. Zur Biofilmbildung kommt es, da die pathogenen Mikroorganismen eine Hülle (Glykokalyx) aus Polysacchariden, Proteinen und Glykoproteinen bilden, an denen wiederum weitere Mikroorganismen anhaften können. So begünstigt ein Dextran-Film die Anhaftung von Streptococcus mutens auf dem Zahnschmelz (M.T. Madigan, J.M. Martinko, P.V. Dunlap, D.P. Clark, Brock: Biology of the Microorganisms, Pearson Benjamin Cummings, 2009, chapter 28, pages 822 ff.). Da bei chronischen Wunden eigentlich immer eine Infektion durch Mikroorganismen vorliegt, kann eine Verwendung von Polysacchariden in einer resorbierbaren Wundauflage die Gefahr einer Biofilm Bildung verstärken bzw. eine erfolgreiche Bekämpfung der Infektion verhindern.

Ein wesentlicher Nachteil der bekannten Polyurethanschäume ist, dass sie sich nicht exakt an das Wundbett anpassen und somit die Wunde nicht ganz vollständig abgedeckt wird. Dies wirkt sich besonders negativ auf die Wundheilung aus, da sich Fibroblasten oder Keratinocyten somit nicht über das gesamte Wundbett an den Polyurethanschaum anhaften können, um die Wundheilung zu beschleunigen.

Ein weiterer Nachteil des in US 2007/0299151 beschriebenen Polyurethanschaums ist zudem, dass dessen Resorptionsgeschwindigkeit nicht einstellbar ist. Vorteilhafterweise sollte eine Wundabdeckung aus Polyurethanschum bei stark exsudierenden, infizierten Wunden schnell resorbierbar sein, so dass der Pflegende eine bessere Kontrolle über den Zustand der Wunde bei jedem Verbandswechsel des Sekundärverbandes erhält. Bei schwach exsudierenden Wunden, bei denen ein häufiger Verbandswechsel nicht nötig ist, sollte ein Polyurethanschaum hingegen eine längere Resorptionszeit aufweisen. So kann die Pflegekraft weiterhin beim Wechsel des Sekundärverbandes den Zustand der Wunde einschätzen und über die weitere Behandlung entscheiden.

Die Aufgabe der vorliegenden Erfindung besteht daher darin die Nachteile des Bekannten zu vermeiden, insbesondere also eine Polyurethanschaum-Wundabdeckung der eingangs genannten Art zu schaffen, die biokompatibel und vom Körper resorbierbar ist; insbesondere soll die Polyurethanschaum-Wundabdeckung verbesserte Fülleigenschaften bei Wunden aufweisen, um einen optimalen Heilungsprozess zu ermöglichen. Diese Aufgabe wird mit einer Polyurethanschaum-Wundabdeckung mit den Merkmalen des Anspruchs 1 gelöst.

Die erfindungsgemässe vom Körper resorbierbare, offenporige Polyurethanschaum-Wundabdeckung, deren Resorptionsgeschwindigkeit einstellbar ist und die sich dem Wundbett durch Quellen bzw. Gelieren anpassen kann, wird hergestellt oder ist herstellbar aus einer Zusammensetzung enthaltend:
a. mindestens eine Polyolkomponente, die mindestens eine Verbindung mit mindestens zwei, insbesondere 2, 3 oder 4 Hydroxygruppen umfasst,
b. mindestens eine Polyisocyanatkomponente oder ein Polyurethanprepolymer, die mindestens eine Verbindung mit mindestens zwei, insbesondere 2, 3 oder 4 Isocyanatgruppen umfasst, und
c. einem Zusatz, welcher mindestens eine der folgenden Substanzen enthält: Polyvinylpyrrolidon, Polyvinylpolypyrrolidon, sulfonierte Copolyester, Polyvinylalkylether, ein Copolymer von Polyvinylpyrrolidon mit Vinylacetat, Vinylimidazol oder Vinylcaprolactam, ein Copolymer von Alkylvinylether mit einer Säure oder einem Anhydrit, insbesondere Maleinsäure oder Maleinanhydrit, sowie Polymere und Copolymere der Acrylsäure und deren Salze oder Mischungen davon.

Die Zusammensetzung zur Herstellung der erfindungsgemässen Polyurethanschaum-Wundabdeckung kann bevorzugterweise noch weitere Komponenten enthalten. Besonders bevorzugt enthält die Zusammensetzung zusätzlich eine oder mehrere der folgenden Komponenten:
d. mindestens einen Porenöffner, vorzugsweise bestehend aus einem divalenten Salz einer langkettigen Fettsäure in Pulverform
e. einen Porenstabilisator, vorzugsweise Türkischrotöl oder Polyethersiloxan
f. mindestens einem Katalysator
g. mindestens ein Polycaprolacton, bevorzugt Poly(ε-Caprolacton)
h. mindestens ein Mono- oder Polysaccharid.

Vorteilhafterweise sollte ein resorbierbarer Polyurethanschaum keine Biofilm begünstigende Polysaccharide enthalten, dennoch aber über eine Komponente verfügen, die die positiven physikochemischen Eigenschaften von Polysacchariden imitieren kann und zugleich die Adhäsion von eukaryotischen Zellen fördert.

Die Resorptionsgeschwindigkeit der Polyurethanschaum-Wundabdeckung lässt sich durch geeignete Wahl des Polyethylenglykols, des Mischverhältnisses der Polyethylenglykole in einer Mischung aus Polyethylenglykolen mit verschiedenen Molmassen, durch die eingesetzte Menge an Poly(ε-Caprolacton) wie auch dem Verhältnis der Menge des Poly(ε-Caprolacton) zur Menge des mindestens einen Polyethylenglykols, dem Zusatz c. und dem Wasseranteil einstellen. Zudem lässt sich dadurch auch die Weichheit der Polyurethanschaum-Wundabdeckung einstellen.

Durch Beifügen von Zusatz c. und durch geeignete Wahl der Polyol-Komponente geliert bzw. quillt der Polyurethanschaum bei Kontakt mit Wundexsudat leicht. Dadurch passt sich der Schaum sehr gut den Konturen des Wundbetts an und dieses wird so vollständig gefüllt. Die neu gebildeten, einwachsenden Fibroblasten, Keratinocyten und Epithelzellen müssen dadurch nicht zunächst die Lücke zwischen Wundrand und Wundfüllmaterial verschliessen, sondern können direkt an den Polyurethanschaum als artifizielle extrazelluläre Matrix anhaften.

Ein weiterer Vorteil besteht auch darin, dass das Quellen zu einer verstärkten Stossdämpfung durch die eingebettete Polyurethanschaum-Wundabdeckung in der Wundhöhle führt.

Als Gelieren wird hier Aufquellen verstanden, ohne dass es dabei zu einer vollständigen Lösung kommt. Dabei nehmen sowohl die Polyole und der Zusatz c. die Feuchtigkeit des Wundexsudats auf und binden diese.

Der Zusatz c., insbesondere das Polyvinylpyrrolidon, wird in der vorliegenden Erfindung in einer geringen Konzentration dem Polyethylenglykol beigemischt, wobei es nach Ablaufen der Reaktion grösstenteils in Form von "Inseln" sowohl auf der Oberfläche wie auch im Innern des Polyurethanschaums verteilt ist. Dadurch ist die Konzentration des Zusatzes c., insbesondere des Polyvinylpyrrolidons, auf dem Schaum partiell erhöht. Dies führt dazu, dass selbst bei Zugabe einer geringen Menge an Zusatz c., insbesondere an Polyvinylpyrrolidon, in der Zusammensetzung es zur Bildung von Bereichen kommt, wo die Konzentration für biologische Aktivität ausreicht.

Ein besonderer Vorteil bei Anwesenheit von Polyvinylpyrrolidon im Zusatz c. ist, dass Polyvinylpyrrolidon Proteine binden kann. Dadurch werden Wachstumsfaktoren an der Diffusion gehindert und freie Matrixmetalloproteasen (MMPs) gebunden, so dass die Konzentration dieser im Bereich der nachwachsenden Zellen absinkt. Dies führt in der Folge zu einer positiven Beeinflussung der Wundheilung.

Überraschend ist zudem, dass durch die Verwendung von Polyvinylpyrrolidon im Polyurethanschaum die Adhäsion und Formierung von bakteriellem Biofilm vermindert wird, es aber gleichzeitig zu einer Adhäsion von eukaryotischen Zellen kommt. Ohne dass die Erfindung auf einen Wirkmechanismus zu beschränken wäre, wird derzeit vermutet, dass für die Wundheilung sich zunächst eine provisorische extrazelluläre Matrix aus Proteinen bilden muss, die als Adhäsionsgrundlage für die Zellen dient. Dies wird vermutlich durch die Anwesenheit von Polyvinylpyrrolidon begünstigt.

Weitere Vorteile bei der Verwendung von Polyvinylpyrrolidon als Ersatzstoff für Polysaccharide im resorbierbaren Polyurethanschaum sind die gute biologische Verträglichkeit und die toxikologische Unbedenklichkeit.

Die Polyolkomponente enthält mindestens eine Verbindung mit mindestens zwei oder mehr Hydroxygruppen. Vorzugsweise besteht die Polyolkomponente aus einem Gemisch von zwei oder mehr Verbindungen mit mindestens zwei oder mehr Hydroxygruppen. Bevorzugte derartige Verbindungen sind dabei hydroxyterminierte Polyether wie z.B. α,ω-Dihydroxypoly(oxyethylene), α,ω-Dihydroxypoly(1,2-ethylenoxid), α,ω-Dihydroxypoly(1,2-propylenoxid), α,ω-Dihydroxypoly(1,3-trimethylenoxid), α,ω- Dihydroxypoly(1,4-tetramethylenoxid), α,ω-Dihydroxypoly(methylenoxy-1,2- ethylenoxid) und dergleichen sowie Copolymere davon, mit Molmassen vorzugsweise bis zu 15000 g/mol, hydroxyterminierte aliphatische Polycarbonate wie z.B. α,ω- Dihydroxypoly(ethylencarbonat), α,ω-Dihydroxypoly(1,2-propylencarbonat), α,ω- Dihydroxypoly(1,3-propylencarbonat), α,ω-Dihydroxypoly(tetramethylencarbonat), α,ω-Dihydroxypoly(hexamethylencarbonat) und dergleichen sowie Copolymere davon, jeweils mit Molmassen vorzugsweise bis zu 15000 g/mol, Polyanhydride aus Dicarbonsäuren, wie z.B. Malonsäure, Bernsteinsäure, Glutarsäure und dergleichen sowie Copolymere daraus, jeweils mit Molmassen vorzugsweise bis zu 15000 g/mol, niedermolekulare zwei oder mehrwertige Alkohole wie z.B. Glykol, Polyethylenglykol, 1,2- Propylenglykol, 1,3-Propylenglykol, Butandiol, Pentandiol, Hexandiol und längerkettige lineare oder verzweigte aliphatische Diole, Glycerin, Triethanolamin, Pentaerythrit, 2,2-Bis(hydroxymethyl)propanol und dergleichen, Hydroxygruppen-enthaltende Aminosäuredimere, - trimere oder -oligomere, z.B. aus Tyrosin und/oder Serin, sowie Zuckeralkohole wie Sorbit und andere Naturstoffe oder Naturstoffderivate mit mindestens zwei Hydroxygruppen und dergleichen. Des Weiteren können Polyestertriole wie Rizinusöl und Türkischrotöl eingesetzt werden.

Bevorzugt werden als Polyol-Komponenten Polyestertriole zugesetzt, die eine höhere Verzweigungsdichte bei der Vernetzung des Polyurethanschaums hervorrufen. Besonders bevorzugt wird Rizinusöl eingesetzt.

Besonders bevorzugt werden in der Polyolkomponente Polyester eingesetzt, deren Endgruppen Hydroxygruppen sind. Beispiele derartiger Verbindungen sind Polycaprolactondiol und Polycaprolactontriol (z.B. unter der Bezeichung Capa kommerziell von Solvay erhältlich). Weitere Beispiele sind α,ω-Dihydroxypoly(D,L-lactid), α,ω-Dihydroxypoly(D-lactid), α,ω-Dihydroxypoly(L-lactid), α,ω-Dihydroxypoly(glycolid), α,ω-Dihydroxypoly (hydroxybutyrat) und andere aliphatische Polyester sowie deren Copolymere einschliesslich segmentierter Blockcopolymere aus Polyether und Polyestersegmenten, wie sie z.B. aus der Umsetzung von hochmolekularen Polyestern mit hydroxyterminierten Poly (alkylenglykolen) erhalten werden können, sowie Mischungen aus derartigen Polyolen.

Besonders bevorzugt werden solche Verbindungen eingesetzt, die bioverträglich und vom Körper resorbierbar sind. Beispielsweise sind dies Poly(ε-Caprolacton) (PCL), Poly(ε-Caprolacton-Co-Glycolid-Co-DL-Lactid), verzweigte sowie unverzweigte Polyethylenglykole (PEG), PCL-b-PEG-b-PCL, (α,ω-Dihydroxy-Oligo(((R)-3-Hydroxybutyrat-Co-(R)-3-Hydroxyvalerat)-Block-Ethylenglykol).

In einer besonders bevorzugten Ausführungsform der Erfindung wird als Polyolkomponente eine Mischung mit mindestens einem Polyethylenglykol und zusätzlich einem Poly(ε-Caprolacton) verwendet. Das mindestens eine Polyethylenglykol kann dabei verzweigt oder unverzweigt sein und eine mittlere molare Masse von 100 bis 15000, bevorzugt von 300 bis 5000, aufweisen. Bevorzugt umfasst die Polyolkomponente eine Mischung aus Polyethylenglykolen mit verschiedenen mittleren molaren Massen. Das mindestens eine Poly(ε-Caprolacton) kann dabei verzweigt oder unverzweigt sein und eine mittlere molare Masse von 100 bis 15000, bevorzugt von 100 bis 1000, aufweisen.

Für die Herstellung des Polyurethanschaums können Polyurethan Prepolymere verwendet werden. Im Allgemeinen weisen die in der vorliegenden Erfindung verwendeten Prepolymere eine molare Masse zwischen 400 und 15000, bevorzugt zwischen 400 und 10000 und besonders bevorzugt zwischen 400 und 1000 auf.

Neben der Verwendung eines Polyurethanprepolymers kann eine Polyisocyanatkomponente mit mindestens einer Verbindung mit mindestens zwei Isocyanatgruppen in der Zusammensetzung verwendet werden. Die Polyisocyanatkomponente enthält dabei vorzugsweise mindestens ein biokompatibles aliphatisches Polyisocyanat oder mindestens eine von einem biokompatiblen Polyamin abgeleitete Verbindung. Bevorzugte Verbindungen sind dabei unter den Folgenden ausgewählt: gegebenenfalls substituierte Alkylendiisocyanate mit 3 bis 12 Kohlenstoffatomen wie Hexamethylendiisocyanat, oder Lysindiisocyanat, gegebenenfalls substituerte Cycloalkylendiisocyanate mit 5 bis 15 Kohlenstoffatomen wie Cyclohexylendiisocyanat, gegebenenfalls substituerte Alkylcycloalkylendiisocyanate mit 6 bis 18 Kohlenstoffatomen wie Isophorondiisocyanat, gegebenenfalls substituierte aromatische Diisocyanate wie p-Phenylendiisocyanant, Toluyldiisocyanate (alle Isomere sowie deren Mischungen), 4,4'-Diphenylmethandiisocyanat, sowie Isomere, Trimere und höhere Oligomere dieser Diisocyanate, Uretdione aus diesen Isocyanaten, Cyanurate und Isocyanurate aus diesen Isocyanaten und dergleichen.

Besonders bevorzugt werden Hexamethylendiisocyanat, 1,6-Diisocyanatohexan (HDI), 1,4-Diisocyanatobutane (BDI), Isophorondiisocyanat (IPDI), Dicyclohexylmethandiisocyanat (H12MDI), Lysinmethylesterdiisocyanat (LDI) oder 4.4'-Diphenylmethandiisocyanat (MDI) eingesetzt.

Als Porenöffner eignen sich vorzugsweise nicht toxische Substanzen, deren Konzentration auch nach Resorption in den Körper nicht toxisch ist, bestehend aus einem divalenten Metallsalz einer langkettigen Fettsäure mit 1 bis 22 Kohlenstoff Atomen. Bevorzugt werden Porenöffner eingesetzt, welche Calciumstearat enthalten. Die bevorzugte Konzentration an Porenöffner in der Zusammensetzung der vorliegenden Erfindung liegt zwischen 0.01 und 5 % Gewichtsprozent.

Die Zusammensetzung kann ferner Schaum-Stabilisatoren enthalten, die vorzugsweise nicht toxisch sind und deren Konzentration auch nach deren vollständiger Resorption durch den Körper keinen toxischen Wert erreicht. Vorzugsweise werden als Stabilisatoren nicht ionische und anionische Tenside eingesetzt. Beispielsweise können als Stabilisatoren ein Polyethersiloxan, ein Salz einer sulfonierten Fettsäure oder ein Salz einer Fettsäure eingesetzt werden. Bevorzugt wird ein Salz einer sulfonierten Fettsäure, besonders bevorzugt Türkischrotöl (sulfoniertes Rizinusöl) verwendet. Die Konzentration der eingesetzten Stablisiatoren liegt in der vorliegenden Erfindung bei 0.001 bis 3% Gewichtsprozent.

Darüber hinaus kann die Zusammensetzung auch nicht toxische Katalysatoren enthalten, deren Konzentration auch nach Resorption in den Körper nicht toxisch ist und die die Reaktion zwischen Hydroxy- und Isocyanatgruppen katalysieren. Bevorzugt wird in der vorliegenden Erfindung ein Organometallischer Katalysator und/oder ein tertiärer Amin-Katalysator verwendet. Besonders bevorzugt werden dabei Bismutkatalysatoren und/oder 2'2'-Dimorpholinyldiethylether. Die Menge an eingesetzten Katalysatoren beträgt insbesondere 0.01 bis 1 Gewichtsprozent.

Im Weiteren können auch noch weitere Komponenten wie Emulgatoren und dergleichen in der Zusammensetzung enthalten sein.

Als Komponente c. wir bevorzugt Polyvinylpyrrolidon zugegeben. Dabei kann das Polyvinylpyrrolidon aus Polyvinylpyrrolidon mit einer bestimmten mittleren molaren Masse oder einer Mischung von Polyvinylpyrrolidon mit verschiedenen mittleren molaren Massen bestehen. Bevorzugt wird Polyvinylpyrrolidon mit einer mittleren molaren Masse von 7'000 bis 1'000'000 oder eine Mischung davon als Dispersion eingesetzt. Besonders bevorzugt wird Polyvinylpyrrolidon mit einer mittleren molaren Masse von 40'000 bis 60'000 eingesetzt (z.B. unter dem Namen Luvitec K30 von BASF erhältlich).

Als Komponente c. können ebenfalls Copolymere aus Vinylpyrrolidon mit Vinylacetat, Vinylimidazol oder Vinylcaprolactam verwendet werden (z.B. unter den Namen Luvitec VA64W, VA64, VPI55K72W und VPC55K65W von BASF erhältlich). Besonders bevorzugt wird ein Copolymer aus Vinylpyrrolidon und Vinylimidazol, da die Imidazolstruktur die Bindung an Proteine fördert.

Die Komponente c. kann auch sulfonierte Copolyester (z.B. unter dem Namen Eastman AQ-Polymere von Eastman erhältlich), Polyvinylalkylether, bevorzugterweise Polyvinylmethylether (unter dem Namen Lutonal M40 100% von BASF erhältlich), oder Polyvinylpolypyrrolidon enthalten.

Weitere bevorzugte Substanzen, die in Komponente c. enthalten sein können sind Copolymere von Alkylvinylether, bevorzugt Methyl- oder Ethylvinylether, mit einer Säure oder einem Anhydrit, bevorzugt Maleinsäure- oder Anhydrit (z.B. unter dem Namen Gantrez AN 169 oder Gantrez S-Reihe bei ISP erhältlich). Weiter bevorzugt enthält Komponente c. Copolymere von Polyacrylat und Natrium-Polyacrylat (beispielsweise unter dem Namen Superabsorber T 5066 F bei Degussa erhältlich).

Bevorzugt enthält die Zusammensetzung, aus der der erfindungsgemässe Polyurethanschaum hergestellt wird oder herstellbar ist, 1 bis 96 Gewichtsprozent, besonders bevorzugt zwischen 30 und 60 Gewichtsprozent und ganz besonders bevorzugt zwischen 35 und 50 Gewichtsprozent der mindestens einen Polyolkomponente.

Bevorzugt enthält die Zusammensetzung weiter zwischen 4 und 60 Gewichtsprozent, besonders bevorzugt zwischen 40 und 60 Gewichtsprozent und ganz besonders bevorzugt zwischen 45 und 55 Gewichtsprozent der mindestens einen Isocyanatkomponente oder Polyurethanprepolymers.

Zusätzlich enthält die Zusammensetzung wenigstens ein Polyvinylpyrrolidon in einer Menge von 0.001 bis 10.0 Gewichtsprozent, bevorzugt zwischen 1.0 und 4.0 Gewichtsprozent. Durch unterschiedliche Konzentrationenverhältnisse an Polyvinylpyrrolidon, Polycaprolacton, Polyethylenglykol und/oder Komponente c. lässt sich die Resorptionsgeschwindigkeit des Schaums einstellen. Bevorzugt lässt sich die Resorptionsgeschwindigkeit zudem durch Zugabe von Wasser in die Zusammensetzung und durch Variieren der Wasserkonzentration in der Zusammensetzung einstellen.

Der in der vorliegenden Erfindung beschriebene Polyurethanschaum dient unter anderem als artifizielle extrazelluläre Matrix für einwachsende Zellen bei Haut- und Bindegewebsverletzungen und ist deshalb vorzugsweise porös. Die Poren können untereinander verbunden sein (offenporige Struktur) aber auch gegeneinander abgetrennt (geschlossenporige Struktur). Besonders bevorzugt weist der Schaum eine offenporige Struktur auf. Der prozentuale Anteil offener Poren beträgt dabei mindestens 70%, besonders bevorzugt mindestens 90%. Im trockenen Zustand weisen die Poren einen durchschnittlichen grössten Durchmesser von 50 bis 600 µm auf, wodurch sowohl die Haut- und Bindegewebszellen, Fibroblasten, Keratinocyten und Epithelzellen einwachsen können, als auch Platz für die Bildung neuer Blutgefässe (Angiogenese) geschaffen wird. Da der in der Erfindung vorliegende Polyurethanschaum bei Kontakt mit Flüssigkeiten, insbesondere Wundexsudat geliert bzw. quillt, weisen die Poren im Schaum im nassen, d.h. mit Flüssigkeit oder Wundexsudat durchtränkten Zustand vorzugsweise einen durchschnittlichen grössten Durchmesser von 100 bis 400 µm auf. Besonders bevorzugt beträgt der durchschnittliche grösste Durchmesser der Poren im nassen Zustand zwischen 100 und 250 µm.

Der erfindungsgemässe Polyurethanschaum wird vorzugsweise als resorbierbare Wundauflage oder Wundfüller verwendet. Durch die gelierenden bzw. quellenden Eigenschaften passt sich der Schaum den Konturen des Wundbetts an. Dadurch kann der Polyurethanschaum als Wundfüller im Sinne einer artifiziellen extrazellulären Matrix verwendet werden, wobei neu gebildete Zellen direkt an den Schaum anhaften können und nicht zunächst die Lücke zwischen Matrix und Wundrand schliessen müssen. Diese Anwendung ist bei den bislang bekannten Polyurethanschäumen, die als Wundabdeckung dienen nicht möglich, da sie nicht resorbierbar sind und deshalb aus dem Wundbett oder der Wundhöhle entfernt werden müssen.

Das im Schaum vorhandene Polyvinylpyrrolidon erleichtert zudem das An- und Einwachsen von Zellen, führt zu einer verstärkten Stossdämpfung, Wachstumsfaktoren werden an der Diffusion gehindert und freie Matrixmetalloproteasen (MMPs) werden gebunden, so dass die Konzentration dieser im Bereich der nachwachsenden Zellen absinkt. Dieser Effekt beruht überwiegend auf der hygroskopischen Eigenschaft der Polyvinylpyrrolidonmolekülen.

Der in der vorliegenden Erfindung beschriebene Polyurethanschaum kann mit einer flüssigen biologisch aktiven Substanz oder einem flüssigem Wirkstoff ausgestattet werden, indem der Schaum zunächst mit einem flüssigen Wirkstoff getränkt und anschliessend getrocknet wird. Dadurch bleibt der Wirkstoff in kristalliner Form an den Porenwänden zurück.

Des Weiteren kann die Oberfläche des in der vorliegenden Erfindung beschriebenen Polyurethanschaums modifiziert werden und mit festen biologisch aktiven Substanzen ausgestattet werden. Ein mögliches Verfahren dazu wäre die Festphasensynthese nach Merrifield. Weitere, dem Fachmann bekannte Verfahren können ebenfalls angewendet werden.

Biologisch aktive Substanzen können dabei Wirkstoffe, Moleküle oder Komponenten sein, die einen biologischen oder chemischen Effekt in der Wunde auslösen. Biologisch aktive Substanzen können dabei synthetische Moleküle, Biomoleküle oder Gemische dieser sein und beinhalten Enzyme, organische Katalysatoren, Ribozyme, Organometalle, Proteine, Glykoproteine, Peptide, Polyaminosäuren, Antikörper, Nukleinsäuren, Steroide, Antibiotika, antivirale und antifugale Substanzen, Analgetika, Immunsuppressiva, Cytokine, Kohlenhydrate, oleophobe Substanzen, Lipide, Komponenten der natürlichen extrazellulären Matrix, Pharmazeutika und Therapeutika. Bevorzugt werden Wirkstoffe, die den Wundheilungsprozess unterstützen wie Antiseptika, Antibiotika, Analgetika, Polysaccharide, Komponenten der natürlichen extrazellulären Matrix und Wachstumsfaktoren. Als Wachstumsfaktoren können dabei TGF-β (transforming growth factor β), IGF-I und -II (insulin-like growth factors 1 and 2), PDGF (plateled derived growth factor), EGF (epidermal growth factor), VEGF (vascular endothelial growth factor), KGF (keratinocyte growth factor), FGF-2 (fibroblast growth factor 2), HGF(hepatocyte growth factor) und weitere. Besonders bevorzugt eingearbeitet wird die Kombination der Wachstumsfaktoren PDGF, VEGF und FGF-2, da diese zusammen die Angiogenese am besten fördern.

Zusätzlich kann der Polyurethanschaum in der vorliegenden Erfindung mit einer Saccharidkomponente, wie Monosaccharide wie Dextrose, Mannose, Mannit, Dulcit, Glukose, Fruktose, Galaktose und dergleichen, Disaccharide wie Maltose, Laktose, Saccharose, Cellobiose und dergleichen; Oligo- und Polysaccharide wie beispielsweise Cellulose, Carboxymethylcellulose, Nanocellulose, reduzierte oxidierte Cellulose, Pektin, Amylopektin, Amylose, Chitin, Chitosan, Alginate und dergleichen sowie Mischungen davon versetzt werden. Besonders bevorzugt wird beim erfindungsgemässen Polyurethanschaum Stärke eingesetzt, da es sich um ein kostengünstiges Polysaccharid handelt.

Besonders bevorzugt wird der erfindungsgemässe, resorbierbare Polyurethanschaum in ausreagierter Form als artifizielle extrazelluläre Matrix für Haut- und Bindegewebsverletzungen verwendet. Dabei kann dieser in Form einer Rolle, einer Scheibe oder in einer sonstigen geeigneten Form als Wundauflage eingesetzt werden. Ganz besonders bevorzugt kann der Polyurethanschaum entsprechend der Grösse der Wunde zugeschnitten werden, z.B. durch eine Pflegefachkraft.

Des Weiteren bevorzugt wird der erfindungsgemässe, resorbierbare Polyurethanschaum in ausreagierter Form als Wundfüller für tiefe Wundhöhlen eingesetzt wie sie beim Dekubitus entstehen können. Dazu kann der Polyurethanschaum in Form einer Spirale oder ähnlichem zurechtgeschnitten und als ein Stück in die Wundhöhle gegeben werden. Der Polyurethanschaum hat auch hier die Funktion einer artifiziellen extrazellulären Matrix.

Bevorzugt wird der resorbierbare Polyurethanschaum auch als Trägermaterial für Zellen bei Transplantaten eingesetzt werden. Besonders bevorzugt werden Haut- und Bindegewebszellen, wie Fibroblasten, Keratinocyten und Epithelzellen, aber auch Mischkulturen aus verschiedenen Zellen. Es können aber auch Stammzellen z.B. aus der Haut oder dem Bindegewebe verwendet werden.

In einer weiteren bevorzugten Ausführungsform der Erfindung weist die Verpackung des Polyurethanschaums eine Messtabelle auf, anhand derer unter Berücksichtigung der Quelleigenschaften des Schaums das Zuschneiden der Wundauflage auf bestimmte Grössen und/oder Formen erleichtert wird.

In einer weiteren Ausführungsform ist der Polyurethanschaum auf eine Trägerfolie, insbesondere einer Polymerfolie appliziert, auf der ein Schnittmuster unter Berücksichtigung der Quelleigenschaften angegeben ist, das das Zuschneiden der Wundauflage auf eine bestimmte Grösse und/oder Form erleichtert.

In einer besonderen Ausführungsform kann der Polyurethanschaum zur lokalen Applikation eines Wirkstoffs verwendet werden. Da der Schaum in den Körper resorbiert wird, erfolgt eine kontinuierliche Abgabe des Wirkstoffs bis der Schaum vollständig abgebaut ist. Besonders geeignet ist die Verwendung des erfindungsgemässen Polyurethanschaums für eine kontinuierliche Abgabe eines Wirkstoffs.

Bevorzugt wird der erfindungsgemässe Polyurethanschaum in einem kontinuierlichen Sprühauftragsverfahren hergestellt. Dabei wird das Reaktionsgemisch mit einem Sprühkopf und unter Druck auf eine Unterlage gesprüht. Die Komponenten werden dabei entweder direkt im Sprühkopf oder kurz davor in einer Mischkammer zusammengemischt. Durch die plötzliche Druckänderung beim Austritt aus dem Sprühkopf wird der austretende Strahl aufgerissen und es entsteht ein Sprühkegel. Zur Expansion des Schaums kann ein zugesetzte Treibmittel (Stickstoff, CO₂, gasförmiger Kohlenwasserstoff, thermisch aktivierbares Treibmittel etc.) oder das bei der Reaktion der Komponenten entstehende CO₂ verwendet werden. Besonders bevorzugt wird der Polyurethanschaum im Sprühauftragsverfahren hergestellt, wobei Wasser als Treibmittel benutzt wird, um eine Porenstruktur zu erhalten. Das bei der Reaktion entstehende CO₂ führt zu der Porenbildung. Der Schaum kann dazu auf ein Förderband gegeben werden oder in einer Form geschäumt werden. Die so entstandenen Polyurethanblöcke werden anschliessend auf die benötigten Dicken, vorzugsweise zwischen 1 und 100mm, besonders bevorzugt zwischen 2 und 20mm, zurechtgeschnitten. Je nach der Menge des in der Zusammensetzung vorhandenen Wassers lässt sich zudem die Grösse und Anzahl der Poren beeinflussen.

Eine weitere bevorzugte Ausführungsform dieser Erfindung betrifft einen Satz ("kit of parts") an Polyurethanschaum-Wundabdeckungen mit verschiedenen Absorbtionsgeschwindigkeiten. Die Absorbtionsgeschwindigkeit der einzelnen Wundabdeckung lässt sich durch die bei der Herstellung eingesetzte Menge an Caprolacton und/oder Polyvinylpyrrolidon einstellen. Bevorzugterweise kann die Resorbtionsgeschwindigkeit auch durch die eingesetzte Menge an Wasser sowie durch Auswahl der Polyolkomponente beeinflusst werden. Durch den erfindungsgemässen Satz an Polyurethanschaum-Wundabdeckungen mit verschiedenen Resorbtionsgeschwindigkeiten kann bei der Wundversorgung jeweils eine Wundabdeckung mit der für den Wundtyp optimalen Resorbtionsgeschwindigkeit ausgwählt werden.

Weitere Vorteile und Einzelmerkmale der Erfindung ergeben sich aus der nachfolgenden Beschreibung von Ausführungsbeispielen und Figuren. Es zeigen:
- Fig. 1:: Vergleich der Abbaurate eines Schaums mit 2% PVP mit der Abbaurate eines Schaums ohne PVP
- Fig. 2:: Vergleich der Abbauraten von Schäumen mit verschiedenen Mischverhältnissen an PEG und Polycaprolacton
- Fig. 3:: Einfluss der Substitution von unverzweigtem mit ver- zweigtem Polycaprolacton unter Variation des Mischver- hältnisses Polycaprolacton/PEG
- Fig. 4:: Quellverhalten von Schäumen mit unterschiedlichen Mischverhältnissen Polycaprolacton/PEG
- Fig. 5:: Vergleich des Quellverhaltens eines erfindungsgemässen Schaums in Wasser und in physiologischer Kochsalzlösung
- Fig. 6:: Einfluss des Wassergehalts in der Zusammensetzung auf die Grösse und Anzahl der Poren
- Fig. 7:: Vergleichsaufnahme zwischen der Porenstruktur eines Schaums im trockenen Zustand und eines Schaums im nas- sen Zustand
- Fig. 8:: Grössenzunahme eines erfindungsgemässen Schaums nach 2 Tagen bei Raumtemperatur in Wasser.

Im Folgenden wurden Messungen zur Abbaurate und dem Quellverhalten von Polyurethanschäumen mit variierenden Konzentrationen an PEG, Polycaprolacton und Polyvinylpyrrolidon durchgeführt. Eine Übersicht der dabei verwendeten Schaum Zusammensetzungen gibt Tabelle 1 (alle Angaben in Gramm):

| **Rohstoff** | **Zusammensetzung** | | | | | | | |
|---|---|---|---|---|---|---|---|---|
| | **A** | **B** | **C** | **D** | **E** | **F** | **G** | **H** |
| PEG-400 | 8 | 8 | 8 | 8 | 8 | 8 | 8 | 8 |
| PEG-4000 | 12 | 0 | 6 | 0 | 6 | 12 | 12 | 12 |
| Sympatens TRH/400 | 0.02 | 0.02 | 0.02 | 0.02 | 0.02 | 0.02 | 0.02 | 0.02 |
| CAPA 2402 | 0 | 12 | 6 | 0 | | 0 | 0 | 0 |
| CAPA 4801 | 0 | 0 | 0 | 12 | 6 | 0 | 0 | 0 |
| Rizinusöl | 4 | 4 | 4 | 4 | 4 | 4 | 4 | 4 |
| Coscat 83 | 0.28 | 0.28 | 0.28 | 0.28 | 0.28 | 0.28 | 0.28 | 0.28 |
| Jeffcat DMDEE | 0.23 | 0.23 | 0.23 | 0.23 | 0.23 | 0.23 | 0.23 | 0.23 |
| PVP | 1.06 | 1.06 | 1.06 | 1.06 | 1.06 | 0 | 1.06 | 1.06 |
| Ca-Stearat | 0.5 | 0.5 | 0.5 | 0.5 | 0.5 | 0.5 | 0.5 | 0.5 |
| Türkischrotöl | 0.1 | 0 | 0 | 0 | 0 | 0.1 | 0.2 | 0.2 |
| Wasser | 0.3 | 0.3 | 0.3 | 0.3 | 0.3 | 0.3 | 0.25 | 0.2 |
| Desmodur E 305 | 26.5 | 26.5 | 26.5 | 26.5 | 26.5 | 26.5 | 24.75 | 23.0 |

Die Herstellung der in den folgenden Beispielen verwendeten Polyurethanschäume erfolgte stets nach dem gleichen Protokoll. Die Offenporigkeit der Schäume wurde durch Wasser als Treibmittel (CO₂-Bildung) und Calciumstearat als Porenöffner gewährleistet. Das PVP wurde, falls in der Zusammensetzung vorhanden, in PEG-400 bei 170°C gelöst. Nachdem das PVP gelöst war, wurden die restlichen Komponenten wie Rizinusöl (Polyestertriol), Sympatens TRH/400 (Kolb AG aus Hedingen, Deutschland) als Emulgator sowie PEG und/oder Polycaprolacton oder Mischungen davon beigemischt und bei 170°C gelöst. Das Gemisch wurde danach in einem Umluftofen auf 70° abgekühlt. In einem nächsten Schritt wurde das Gemisch mit Wasser, Coscat 83 (Vertellus Inc., Indianapolis, USA) als Katalysator, Jeffcat (Huntsman Performance Products, The Woodlands, Texas, USA) als Schaumkatalysator, Calciumstearat (Porenöffner) und mit Türkischrotöl als weiterem Emulgator versetzt. Das Gemisch wurde mit auf 70°C vorgewärmtem Desmodur E 305 (Bayer Material Science AG, Leverkusen, Deutschland) versetzt und während 1 Minute intensiv vermischt (in einem PP 200-Becher mit einem SpeedMixer^{™}-Mischer). Der Becher wurde anschliessend verschlossen während 15 Minuten (mit kleinem Loch für Druckausgleich) in einen Heizschrank mit 110° Heiztemperatur gestellt. Danach wurde, damit die Reaktion bis zur Vollständigkeit ablief, der Becher während 3 Stunden in einen Umluftofen bei 70°C gestellt. Nach Abkühlen auf Raumtemperatur wurde der Schaumstoffblock entformt und zurechtgeschnitten.

Zur Bestimmung der Abbaurate wurden die Massenverluste berechnet. Dazu wurde ein Protokoll zur oxidativen Degradation von Polymeren in medizinischen Produkten der ISO-Norm 10993-13:1999 verwendet und eine 3%-ige Wasserstoffperoxid-Lösung als Medium verwendet. Dazu wurden die Prüfkörper unter Vakuum getrocknet bis sie ein stabiles Gewicht zeigten. Die Prüfkörper wurden danach in eine Petrischale mit 3%-iger Wasserstoffperoxidlösung gegeben und bei 37°C inkubiert. Während des Tests wurden die Proben auf einem Laborschüttler konstant bewegt. Zu verschiedenen Zeitpunkten wurden die Prüfkörper bzw. die Reste der Prüfkörper bis zu einem stabilen Gewicht unter Vakuum getrocknet. Aus dem gemessenen Gewicht konnte der Massenverlust berechnet werden. Als Positivkontrollen wurden einerseits die resorbierbare Wundauflage "Promogran Prisma" (Johnson & Johnson), die nach Herstellerangeben eine *in vivo* Resorptionszeit von 2 - 3 Tagen aufweist und andererseits Wundfäden "Monocryl ungefärbt" (Johnson & Johnson), die laut Herstellerangaben eine *in vivo* Resorptionszeit von 90 - 120 Tagen haben, verwendet. Als Negativkontrolle diente der nicht resorbierbare Schaum Corpura MCF 0.3 (Corpura).

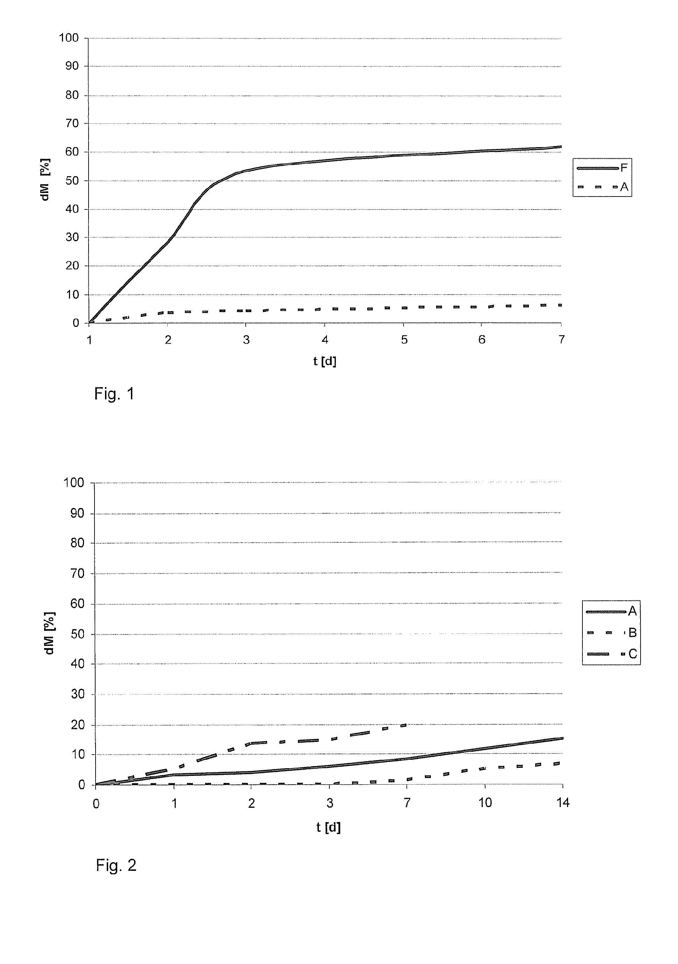

Figur 1 zeigt ein Diagramm mit den Abbauraten eines Schaums mit 2 Gew.-% PVP (Zusammensetzung A) sowie eines Schaums ohne PVP (Zusammensetzung F). Aufgetragen ist der Masseverlust dM des Schaums in % der Anfangsmasse gegen die Zeit t in Tagen. Beide Zusammensetzungen enthalten kein Polycaprolacton. Es zeigt sich, dass ein Polyurethanschaum ohne PVP schneller abgebaut wird als ein Schaum mit PVP. Das PVP dient demnach nicht nur dem Gelieren des Schaums, sondern kann durchaus auch zum Einstellen der Abbaugeschwindigkeit eingesetzt werden.
Figur 2 zeigt ein Diagramm mit den Abbauraten eines Schaums mit 22.7 Gew.-% (12g) PEG-4000 (Kolb AG, Hedingen, Deutschland) gemäss Zusammensetzung A, eines Schaumes mit 22.7 Gew.-% unverzweigten Polycaprolactons mit einer mittleren molaren Masse von 4000 (CAPA 2402 von Perstrop UK Limited, Warrington, UK) gemäss Zusammensetzung B sowie eines Schaums mit je 11.35 Gew.-% PEG-4000 und CAPA 2402 gemäss Zusammensetzung C. In allen Zusammensetzungen sind 15 Gew.-% (8g) an PEG-400 (Kolb AG, Hedingen, Deutschland) vorhanden. Auch hier zeigt sich, dass die Abbaurate eines erfindungsgemässen Polyurethanschaums sich durch Wahl des Verhältnisses zwischen PEG und Polycaprolacton einstellen lässt. An der Figur lässt sich schön sehen, dass Polyurethanschäume mit einer Mischung von PEG mit Polycaprolacton (Zusammensetzung C) schneller abgebaut werden als Schäume mit nur PEG (Zusammensetzung A) oder mit nur Polycaprolacton (Zusammensetzung B).

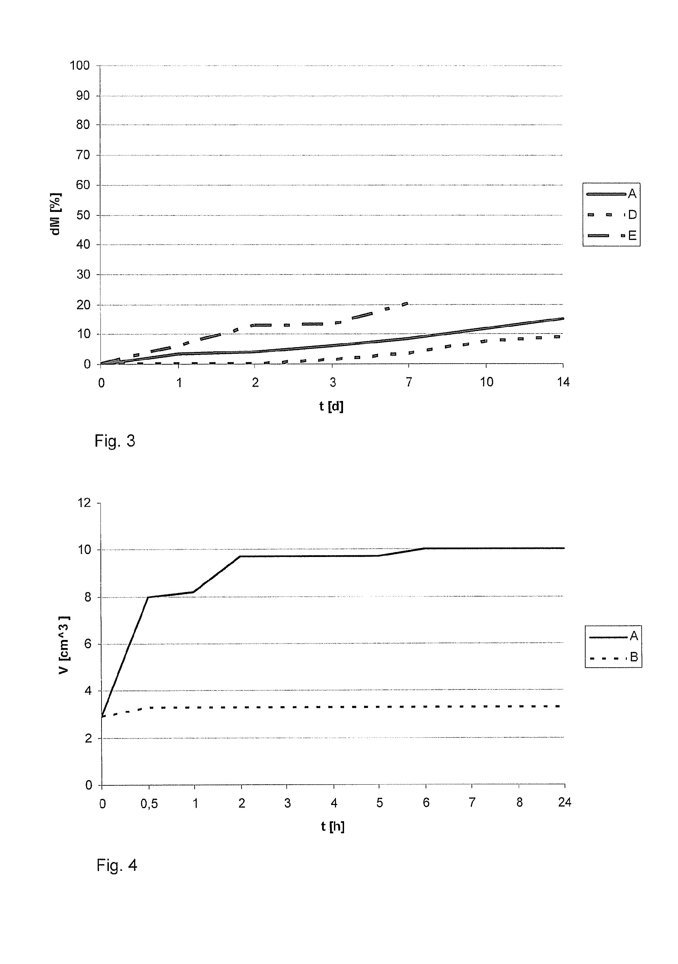

Figur 3 zeigt ein Diagramm mit den Abbauraten eines Schaums mit 22.7 Gew.-% (12g) PEG-4000 (Kolb AG, Hedingen, Deutschland) gemäss Zusammensetzung A, eines Schaums mit 22.7 Gew.-% tetrafunktionalem Polycaprolacton mit einer mittleren molaren Masse von 8000 (CAPA 4801 von Perstrop UK Limited, Warrington, UK) gemäss Zusammensetzung D sowie eines Schaums mit je 11.35 Gew.-% PEG-4000 und CAPA 4801 gemäss Zusammensetzung E. In allen Zusammensetzungen sind 15 Gew.-% (8g) an PEG-400 (Kolb AG, Hedingen, Deutschland) vorhanden. Es zeigt sich, dass die Abbaurate eines erfindungsgemässen Polyurethanschaums sich durch Wahl des Verhältnisses zwischen PEG und Polycaprolacton einstellen lässt.
In Figur 4 ist ein Diagramm zu sehen, welches das Quellverhalten von zwei verschiedenen erfindungsgemässen Polyurethanschäumen darstellt. Zur Bestimmung des Quellverhaltens wurden exemplarisch die Volumenzunahmen von Schäumen, die mit den Zusammensetzungen A und B erhalten wurden, gemessen. Dazu wurden die Testkörper der Schäume zunächst unter Vakuum bis zu einem konstanten Gewicht getrocknet und anschliessend für 24 Stunden in physiologischer Kochsalzlösung gegeben. Das Volumen V wurde in cm³ gemessen. Wie in der Figur deutlich zu sehen ist, führt der Austausch von PEG-4000 mit linearem Polycaprolacton CAPA 2402 dazu, dass der Schaum weniger an Volumen zunehmen kann. Es ist daher nicht nur möglich, die Abbaugeschwindigkeit des Schaums, sondern auch dessen Quellverhalten durch gezielte Auswahl des Mischverhältnissen PEG/Polycaprolacton zu beeinflussen.

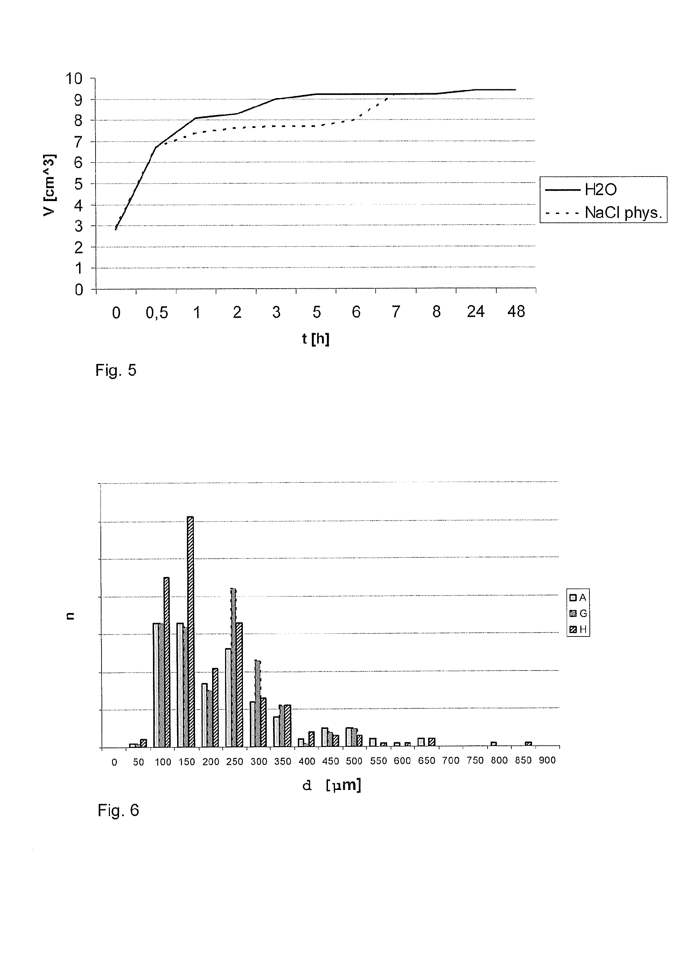

Figur 5 zeigt das Quellverhalten von erfindungsgemässen Schäumen in Wasser und physiologischer Kochsalzlösung, hier am Beispiel eines Schaums mit Zusammensetzung G. Es zeigt sich dabei, dass die Änderung des Volumens V (in cm³) im Wesentlichen unabhängig vom verwendeten Medium (Wasser oder physiologische Kochsalzlösung) ist.
Figur 6 stellt dar, wie sich der Anteil an Wasser in der Zusammensetzung auf die Grösse und Anzahl der Poren des Schaums auswirkt. Zur Analyse der Porengrösse wurden Bilder von Testkörpern von Schäumen mit den Zusammensetzungen A, G und H im trockenen und nassen Zustand mit einem Stereomikroskop Wild M3Z (Wild Leitz) mit einer Kaltlichtquelle Intralux 4000 (Wild Leitz) und einer darauf montierten Digitalkamera aufgenommen, als Maßstab diente ein Linear. Die Durchmesser d der Poren wurden in einem Abschnitt von 20,5 mm² der Testkörper ausgemessen. Dargestellt ist dabei die Verteilung der Porengrössen im nassen Zustand. Im Diagramm wurde die absolute Anzahl n der Poren in Grössenintervallen von 50µm aufgetragen. Dabei zeigte sich, dass sich durch die eingesetzte Menge des Treibmittels Wasser die Porengrösse in den Schäumen variieren lässt. Durch einen geringeren Wasseranteil in den Zusammensetzungen G und H erhöht sich der Anteil der Poren, die im nassen Zustand des Schaums die gewünschte durchschnittliche Grösse von 200 bis 250 µm aufweisen. Da der Polyurethanschaum als resorbierbare Wundauflage später dem Wundexsudat ausgesetzt wird, ist die Porengrösse der Schäume im nassen Zustand entscheidend für die Wundheilung. Dabei sollten die Poren einen durchschnittlichen grössten Durchmesser von 50 und 450 µm, optimal um 200 bis 250 µm aufweisen, so dass Haut- und Bindegewebszellen optimal einwachsen können.

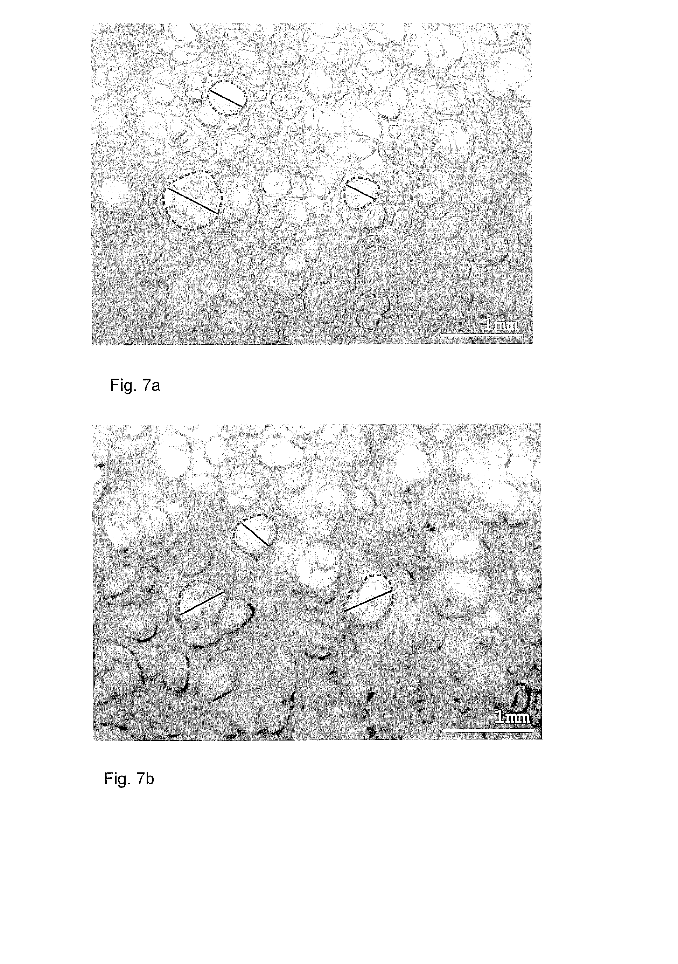

Figur 7 zeigt einen Vergleich zwischen der Porenstruktur eines Schaums im trockenen Zustand (Fig. 7a) sowie der Porenstruktur eines Schaums im nassen Zustand (Fig. 7b). Der Schaum wurde mit der Zusammensetzung 7 hergestellt. Beispielhaft sind auf dieser Figur drei Poren mit einer gestrichelten Linie markiert. Weiter ist bei jeder der markierten Poren beispielhaft der grösste Durchmesser durch eine Linie dargestellt.

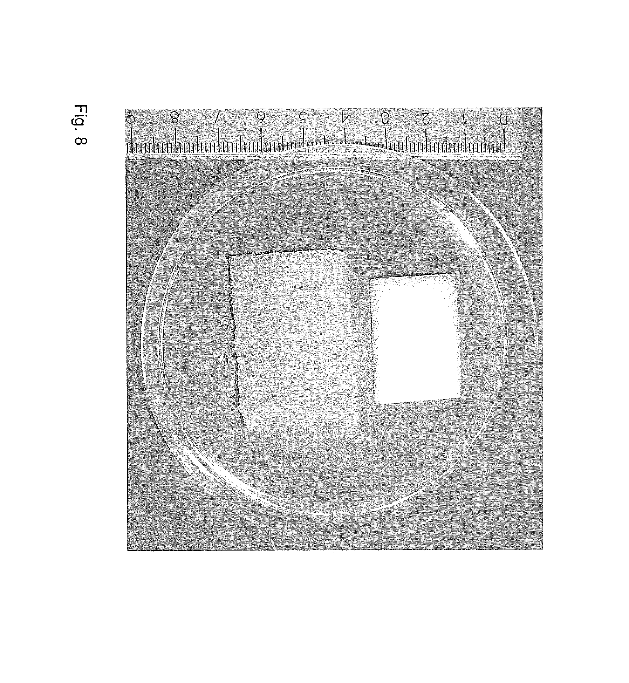

Figur 8 zeigt zwei Stücke eines erfindungsgemässen Schaums gleicher Zusammenstzung. Das Schaumstück oben ist trocken während das Schaumstück unten im nassen Zustand vorliegt. Die Ausgangsgrösse beider Schaumstücke war identisch. Deutlich ist auch hier zu sehen, wie die erfindungsgemässen Schäume durch Kontakt mit Flüssigkeiten aufquellen.

## Patentansprüche

1. Polyurethanschaum-Wundabdeckung hergestellt oder herstellbar aus einer Zusammensetzung enthaltend
a. mindestens eine Polyolkomponente, die mindestens eine Verbindung mit mindestens zwei, insbesondere 2, 3 oder 4 Hydroxygruppen umfasst,
b. mindestens eine Polyisocyanatkomponente oder ein Polyurethanprepolymer, die mindestens eine Verbindung mit mindestens zwei, insbesondere 2, 3 oder 4 Isocyanatgruppen umfasst, und
c. einem Zusatz, welcher mindestens eine der folgenden Substanzen enthält: Polyvinylpyrrolidon, Polyvinylpolypyrrolidon, sulfonierte Copolyester, Polyvinylalkylether, ein Copolymer von Polyvinylpyrrolidon mit Vinylacetat, Vinylimidazol oder Vinylcaprolactam, ein Copolymer von Alkylvinylether mit einer Säure oder einem Anhydrit, insbesondere Maleinsäure oder Maleinanhydrit, sowie Polymere und Copolymere der Acrylsäure und deren Salze oder Mischungen davon.

2. Polyurethanschaum-Wundabdeckung nach Anspruch 1, **dadurch gekennzeichnet, dass** die Zusammensetzung zusätzlich eine oder mehrere der folgenden Komponenten enthält:
d. mindestens einen Porenöffner, vorzugsweise bestehend aus einem divalenten Salz einer Fettsäure, insbesondere in Pulverform
e. einen Porenstabilisator, vorzugsweise Türkischrotöl oder Polyethersiloxan
f. mindestens einen Katalysator
g. mindestens ein Polycaprolacton, bevorzugt Poly(ε-Caprolacton)
h. mindestens ein Mono- oder Polysaccharid, insbesondere Stärke.

3. Polyurethanschaum-Wundabdeckung nach einem der Ansprüche 1 oder 2, **dadurch gekennzeichnet, dass** die mindestens eine Polyolkomponente in einer Menge von 1 bis 96 Gew.-%, bevorzugt zwischen 30 und 60 Gew.-%, besonders bevorzugt zwischen 35 und 50 Gew.-%, enthalten ist.

4. Polyurethanschaum-Wundabdeckung nach einem der Ansprüche 1 bis 3, **dadurch gekennzeichnet, dass** die mindestens eine Polyisocyanatkomponente oder das Polyurethanprepolymer in einer Menge von 4 bis 60 Gew.-%, bevorzugt zwischen 40 und 60 Gew.-%, besonders bevorzugt zwischen 45 und 55 Gew.-%, enthalten ist.

5. Polyurethanschaum-Wundabdeckung nach einem der Ansprüche 1 bis 4, **dadurch gekennzeichnet, dass** der Zusatz c. in einer Menge von 0,001 bis 10 Gew.-%, bevorzugt zwischen 1 und 4 Gew.-%, enthalten ist.

6. Polyurethanschaum-Wundabdeckung nach einem der Ansprüche 1 bis 5, **dadurch gekennzeichnet, dass** das Polyvinylpyrrolidon ein Molekulargewicht von 7'000 bis 1'000'000 g/mol, bevorzugt von 40'000 bis 60'000 g/mol aufweist.

7. Polyurethanschaum-Wundabdeckung nach einem der Ansprüche 1 bis 6, **dadurch gekennzeichnet, dass** die mindestens eine Polyolkomponente mindestens ein Polyethylenglykol enthält.

8. Polyurethanschaum-Wundabdeckung nach einem der Ansprüche 1 bis 7, **dadurch gekennzeichnet, dass** die mindestens eine Polyolkomponente mindestens ein Polycaprolacton, bevorzugt Poly(ε-Caprolacton) enthält.

9. Polyurethanschaum-Wundabdeckung nach einem der Ansprüche 1 bis 8, **dadurch gekennzeichnet, dass** der Schaum im Wesentlichen offene Poren, insbesondere mit einem durchschnittlichen grössten Durchmesser im Bereich von 50-400 µm im nassen Zustand aufweist.

10. Polyurethanschaum-Wundabdeckung nach einem der Ansprüche 1 bis 9, **dadurch gekennzeichnet, dass** der Schaum eine biologisch aktive Substanz enthält, die insbesondere in kristalliner Form vorliegt.

11. Polyurethanschaum-Wundabdeckung nach einem der Ansprüche 1 bis 10, **dadurch gekennzeichnet, dass** ein Schnittmuster aufgebracht ist, welches das Zuschneiden der Polyurethanschaum-Wundabdeckung auf vorbestimmte Grössen und Formen unter Berücksichtigung des Quellverhaltens ermöglicht.

12. Verfahren zur Herstellung einer Polyurethanschaum-Wundabdeckung nach einem der Ansprüche 1 bis 11, **dadurch gekennzeichnet, dass** der Schaum durch ein Sprühauftragsverfahren mit anschliessender Expansion hergestellt wird.

13. Verfahren zur Herstellung einer resorbierbaren Polyurethanschaum-Wundabdeckung nach einem der Ansprüche 1 bis 12, **dadurch gekennzeichnet, dass** der Schaum in einem kontinuierlichen Sprüh-Auftragsverfahren hergestellt wird.

14. Verfahren zur Herstellung einer resorbierbaren Polyurethanschaum-Wundabdeckung nach einem der Ansprüche 1 bis 13, wobei der Schaum in einer Form geschäumt wird.

15. Satz mit Polyurethanschaum-Wundabdeckungen mit verschiedenen Resorbtionsgeschwindigkeiten, **dadurch gekennzeichnet, dass** die Resorbtionsgeschwindigkeit der Wundabdeckungen durch die eingesetzte Menge an Polycaprolacton und/oder Zusatz c. eingestellt ist.
